Europäisches Patentamt

European Patent Office

Office européen des brevets

(19)

(11) Numéro de publication: **0 036 805**
**B1**

(12) **FASCICULE DE BREVET EUROPEEN**

(45) Date de publication du fascicule du brevet:
10.07.85

(51) Int. Cl.⁴: **A 61 F 5/47**

(21) Numéro de dépôt: **81400400.8**

(22) Date de dépôt: **16.03.81**

(54) **Dispositif contraceptif destiné à être placé dans la cavité utérine.**

(30) Priorité: **17.03.80 FR 8005919**

(43) Date de publication de la demande:
**30.09.81 Bulletin 81/39**

(45) Mention de la délivrance du brevet:
**10.07.85 Bulletin 85/28**

(84) Etats contractants désignés:
**AT BE CH DE GB IT LI NL SE**

(56) Documents cités:
**FR - A - 2 165 714**
**GB - A - 2 010 676**
**US - A - 3 438 369**
**US - A - 3 881 475**
**US - A - 3 993 072**
**US - A - 4 054 131**

(73) Titulaire: **ARTS ET TECHNIQUES NOUVELLES Société à responsabilité limitée dite:, 156, rue Oberkampf, F-75011 Paris (FR)**

(72) Inventeur: **Augros, Jacques, Chemin de la Croix Bailliet, F-95400 Villiers le Bel (FR)**

(74) Mandataire: **Hasenrader, Hubert et al, Cabinet BEAU DE LOMENIE 55, rue d'Amsterdam, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

## Description

La présente invention concerne un dispositif contraceptif destiné à être placé dans cavité utérine comportant: une tige axiale et une partie de retenue tridimensionelle, constituée en matériau souple et élastique, cette partie de retenue étant fixée à une extrémité de la tige, et s'étendant transversalement à l'axe de la tige de manière à pouvoir prendre appui sur les parois latérales utérines, ladite partie de retenue comprendant deux boucles fermées fixées chacune par une fixation élastique à l'extrémité de la tige et présentant une symétrie axiale par rapport à l'axe de la tige, ces boucles prolongeant la tige de manière divergente.

Le document US-A-3 438 369 fait connaître, parmi de multiples variantes, un dispositif du type précité. Cependant, les deux boucles fermées se trouvent en vis-à-vis (elles sont symétriques par rapport à un plan). Comme par ailleurs, les boucles sont, globalement, relativement indéformables, un tel dispositif permet une adpatation aux parois utérines seulement dans une direction perpendiculaire au plan de symétrie des boucles, mais non dans une direction transversale du dispositif (parallèle audit plan).

Ce résultat de double adaptabilité est déjà connu, en soi, par le document US-A-3 881 475, mais dans le cadre, inacceptable au point de vue sécurité, d'éléments de retenue ouverts, risquant de perforer la paroi utérine et d'entraîner des saignements.

Obtenir un même résultat avec des boucles fermées est une affaiere entièrement différente.

En effet, selon le document US-A-3 881 475, l'adaptabilité résulte uniquement de la déformation des éléments de retenue eux-mêmes, que se comportent presque comme des ressorts spirales à très faiblé raideur.

Or dans la présente invention, et du fait qu'il s'agit de boucles fermées, celles-ci sont sensiblement indéformables en elles-mêmes. Aussi il est nécessaire de trouver une autre solution pour résoudre le problème de la double adaptabilité transversale.

A noter rapidement que, en sortant du cadre des dispositifs du type défini en tête de ce mémoire, le document US-A-4 054 131 fait connaître un dispositif contraceptif intra-utérin comportant une tige centrale surmontée d'un arc transversal concave vers la tige. Les deux extrémités de l'arc se terminent part de courts bras placés, éventuellement mais non obligatoirement, en dehors du plan commun de l'arc et de la tige. Les deux bras dont il est reconnu qu'ils pourraient perforer le tissu utérin sont munis de spatules afin de conjurer de risque. Cependant, il s'est avéré que tout danger de saignement de la paroi utérine n'était pas écarté.

Le document FR-A-2 165 714 montre un dispositif de conception entièrement différente et compliquée comportant une boucle plane, donc non tridimensionnelle, différentes tiges repliées, et une aspérité en forme de disque.

L'invention a pour but de réaliser un dispositif du type précité en tête de ce mémoire qui soit bien toléré par la femme le recevant, qui soit de construction simple et qui ne présente pas les inconvénients des dispositifs connus.

A cet effet, le dispositif selon l'invention est caractérisé en ce que les boucles ne présentent pas de symétrie par rapport à un plan, mais sont au contraire déportées latéralement en sens opposé l'une par rapport à l'autre et également par rapport à l'axe de la tige, moyennant quoi ladite partie de retenue est adaptable dans ses deux dimensions transversales aux dimensions de la cavité utérine, l'ajustement de l'encombrement transversal du dispositif aux dimensions utérines se faisant par recouvrement partiel des boucles à la manière des lames d'un ciseau.

Du fait de son application dans la dimension transversale de la cavité utérine, où les parois sont moins innervées, le dispositif selon l'invention offre relativement peu de risques de rejet.

Avantageusement, chaque boucle de la partie de retenue présente une forme sensiblement triangulaire s'amincissant dans le sens de l'éloignement de l'axe de la tige.

Avantageusement, la tige et la partie de retenue sont moulées d'une seule pièce en une matière plastique souple et élastique, par exemple appartenant à la famille polyéthylène.

Avantageusement, la tige présente une longueur telle que, le dispositif étant placé dans la cavité utérine, cette tige se trouve en totalité à l'intérieur de cette cavité.

Avantageusement, un produit actif est incorporé à la tige.

Avantageusement, la tige est creuse et fermée, elle contient un produit actif et sa paroi est suffisamment mince pour laisser diffuser à l'extérieur ledit produit actif.

Avantageusement, la tige est réalisée en matière plastique cellulaire.

Il est certain que les documents GB-A-2 010 676 et US-A-3 993 072 font connaître respectivement une paroi perméable et une paroi cellulaire autorisant l'emploi d'un produit actif dans un dispositif contraceptif, mais ils ne proposent pas de dispositif conforme à l'invention dont la structure particulière permet un accrochage efficace dans la cavité utérine.

Les caractéristiques et avantages de l'invention seront mieux compris à la lecture de la description qui va suivre d'un mode de réalisation de l'invention, description faite en référence aux dessins annexés dans lesquels:

— la figure 1 est une vue en élévation d'un dispositif selon un premier mode de réalisation de l'invention;

— la figure 2 est une vue latérale en élévation de la figure 1;

— la figure 3 est une vue de dessus de la figure 1, et

— la figure 4 est une coupe à travers une cavité utérine du dispositif de la figure 1.

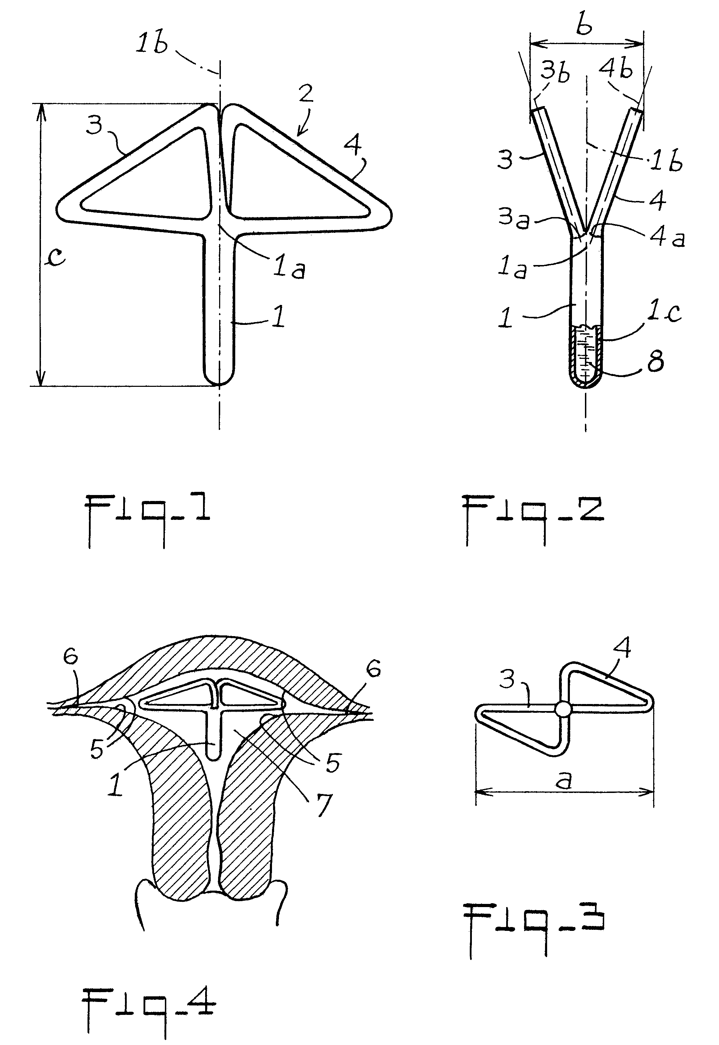

Le dispositif contraceptif représenté comporte (figures 1 et 2) une tige axiale 1 et une partie, dite de retenue 2, constituée en matériau souple. Cette partie de retenue 2 est fixée à une extrémité la de la tige 1.

La partie de retenue 2 est constituée par deux boucles 3 et 4 fixées chacune en un endorit 3a, 4a à l'extrémité 1a de la tige 1. La partie de retenue 2 est tridimensionnelle, et elle s'étend transversalement à l'axe 1b de la tige 1 de manière à prendre appui sur les zone de paroi utérine 5 adjacentes aux trompes 6 (figure 4).

Les boucles 3 et 4 s'étendent à partir de l'axe 1b de la tige 1 dans des directions sensiblement opposées l'une de l'autre; de plus, ces boucles 3, 4 sont sensiblement planes et leurs plans 3b, 4b sont distincts l'un le l'autre.

Les boucles 3, 4 formant la partie de retenue 2 présentent chacune une forme sensiblement triangulaire rectangle s'amincissant dans le sens de l'éloignement de l'axe 1b de la tige 1. Le point de fixation 3a, 4a des boucles 3, 4 est le sommet du triangle rectangle opposé à l'hypothénuse de ce triangle.

Les plans moyens 3b, 4b des boucles 3, 4 divergent l'un de l'autre à partir de l'extrémité 1a de la tige 1. Ces plans moyens 3b, 4b sont symétriques l'un de l'autre par rapport à l'axe 1b de la tige 1 (figure 2), mais n'offrent pas de symètrie plane.

La tige 1 et la partie de retenue 2 sont avantageusement moulées d'une seule pièce en une matière plastique souple, de préférence appartenant à la famille polyéthylène.

La tige 1 présente une longueur telle que, le dispositif étant placé dans la cavité utérine 7, cette tige se trouve en totalité à l'intérieur de cette cavité (figure 4).

Avantageusement, un produit actif est incorporé à la tige 1. Par exemple, la tige 1 peut être creuse et fermée, elle peut contenir un produit actif et sa paroi peut être suffisamment mince pour laisser diffuser à l'extérieur ledit produit actif. Selon un autre exemple, la tige 1 est réalisé en matière plastique cellulaire et celle contient un produit actif remplissant chaque cellule.

Avantageusement, la tige 1 est réalisé en un copolymère d'acétate de vinyl et d'éthylène. Ce materiau a l'avantage de constituer une membrane perméable à des molécules organiques relativement grosses.

Le dispositif intra-utérin qui vient d'être décrit est d'une forme tridimensionnelle effective, et il est conçu et dimensionné dans le sens transversal à la tige 1 de manière à pouvoir être en appui constant sur les faces de la cavité utérine, suivant les modifications cycliques des parois ou les dimensions anatomiques de la cavité. Sa longueur »a« est telle que le dispositif prend appui sur les faces latérales extrêmes 5 opposées.

Les deux autres dimensions »b« et »c« sont adaptées à celle de la vacité (7) où le dispositif est placé, respectivement largeur et hauteur.

Ce dispositif n'est pas susceptible de perforer ou de s'incruster dans la paroi utérine, du fait de la forme à angles arrondis de ses boucles 3, 4 de leur souplesse et de leurs possibilités, importantes de déformation.

Formé par les deux boucles symétriques 3, 4 et la tige axiale 1, ce dispositif occupe les trois zones où se produisent normalement les implantations.

Grâce à l'élasticité de la structure au niveau du rattachement des boucles à la tige centrale, lequel point de rattachement sert pratiquement d'articulation pour les boucles, les deux boucles 3, 4 peuvent se rétracter l'une vers l'autre dans le sens de la longueur »a« et s'adapter ainsi à la longueur des cornes utérines même en cas de disymétrie de ces dernières.

La tige centrale 1 assure la stabilité du dispositif mis en place dans la cavité7, en évitant le basculement des deux boucles 3, 4 autour du centre du dispositif constitué par les points 3a et 4a qui coïncident sensiblement l'un avec l'autre et avec l'extrémité 1a de la tige 1.

Selon l'exemple représenté à la figure 2, la tige 1 est creuse. Sa paroi 1c est d'épaisseur suffisamment fine pour permettre la diffusion, à l'extérieur de la tige, des produits actifs 8 complémentaires qu'elles contient (produits biologiques, chimiques, antibiotiques, etc.).

La tige 1 fait partie intégrante du dispositif intra-utérin et elle peut être moulée avec les boucles 3, 4 ou y être rapportée, si sa matière constitutive est différente de celle des boucles 3, 4. La matière constitutive de la tige 1 peut être une matière alvéolaire imprégnée du produit actif.

Bien entendu, la tige 1 peut être pleine et recevoir un fil métallique en cuivre ou bimétallique, ou bien une gaine imprégnée de produits à action complémentaire.

Dans le cas de la figure 2, l'extrémité de la tige tubulaire 1 peut être obturée par écrasement, soudure ou un moyen mécanique.

Les parois 1c de ce tube peuvent comporter des orifices permettant la diffusion des produits 8 qu'il contient ou faciliter le passage dans le sens allant du milieu physiologique de l'utérus vers l'intérieur du tube 1.

L'ensemble du dispositif intra-utérin peut être moulé dans une matière plastique cellulaire permettant son imprégnation sous-vide par les produits actifs déjà cités. Ces produits peuvent aussi être incorporés à la matière plastique lors de son moulage.

Un fil de traction, non représenté, permettant le retrait du dispositif est accroché à la tige 1.

La souplesse et la forme du dispositif présentement décrit lui permettent de rentrer dans un tube introducteur classique de petit diamètre et facilitent ainsi sa mise en place par des moyens conventionnels.

## Revendications

1. Dispositif contraceptif destiné à être placé dans la cavité utérine, du type comportant une tige axiale (1) et une partie de retenue tridimensionnelle (2) constituée en matériau souple élastique, cette partie de retenue étant fixée à une extrémité (1a) de la tige (1), et s'étendant transversalement à l'axe (1b) de la tige (1) de manière à pouvoir prendre appui sur les parois latérales utérines, ladite partie de retenue (2) comprenant deux boucles fermées (3, 4) fixées chacune par une fixation élastique à l'extrémité (1a) de la tige (1) et présentant une symétrie axiale par rapport à l'axe de la tige, ces boucles prolongeant la tige (1) de manière divergente, caractérisé en ce que les boucles ne présentent pas de symétrie par rapport à un plan, mais sont au contraire déportées latéralement en sens opposé l'une par rapport à l'autre et également par rapport à l'axe de la tige, moyennant quoi ladite partie de retenue est adaptable dans ses deux dimensions transversales (a et b) aux dimensions de la cavité utérine, l'ajustement de l'encrombrement transversal (a) du dispositif auc dimensions utérines se faisant par recouvrement partiel des boucles à la manière des lames d'un ciseau.

2. Dispositif selon la revendication 1, caractérisé en ce que chaque boucle (3 et 4) de la partie de retenue (2) présente une forme sensiblement triangulaire s'amincissant dans le sens de l'éloignement de l'axe (1b) de la tige (1).

3. Dispositif selon l'une quelconque des revendications 1 ou 2, caractérisé en ce que la tige (1) et la partie de retenue (2) sont moulés d'une seule pièce en une matière plastique souple et élastique, par exemple appartenant à la famille polyéthylène.

4. Dispositif selon l'une quelconque des revendications 1 à 3, caractérisé en ce que la tige (1) présente une longueur telle que, le dispositif étant placé dans la cavité utérine (7), cette tige (1) se trouve en totalité à l'intérieur de cette cavité.

5. Dispositif selon la revendication 1, caractérisé en ce qu'un produit actif est incorporé à la tige (1).

6. Dispositif selon la revendication 5, caractérisé en ce que la tige (1) est creuse et fermée, elle contient un produit actif (8) et sa paroi est suffisamment mince pour laisser diffuser à l'extérieur ledit poduit actif.

7. Dispositif selon la revendication 6, caractérisé en ce que la tige est réalisée en un matériau cellulaire.

## Patentansprüche

1. Empfängsnisverhütende Vorrichtung zur Anordnung in der Gebärmutterhöhle, mit einer axialen Stange (1) und einem dreidimensionalen Rückhalteteil (2) aus einem flexiblen und elastischen Material, welcher Rückhalteteil an einem Ende (1a) der Stange (1) fixiert ist und sich derart quer zur Achse (1b) der Stange (1) erstreckt, daß er an den Seitenwänden der Gebärmutter zur Abstützung gelangt, wobei der Rückhalteteil (2) zwei geschlossene Schleifen (3, 4) umfaßt, die jeweils durch eine elastische Fixierung am Ende (1a) der Stange (1) fixiert sind und gegenüber der Achse der Stange eine axiale Symmetrie aufweisen, wobei die Schleifen die Stange (1) divergierend verlängern, dadurch gekennzeichnet, daß die Schleifen bezüglich einer Ebene keine Symmetrie aufweisen, sondern im Gegenteil in zueinander entgegengesetztem Sinn und auch in bezug auf die Achse der Stange seitlich verschoben sind, mit der Maßgabe, daß der Rückhalteteil in seinen beiden Querdimensionen (a und b) an die Dimensionen der Gebärmutterhöhle anpaßbar ist, wobei die Ausrichtung der Querabmessung (a) der Vorrichtung an die Dimensionen der Gebärmutter durch teilweises Überdecken der Schleifen nach der Art einer Schere erfolgt.

2. Vorrichtung nach Anspruch 1, dadurch gekennzeichnet, daß jede Schleife (3, 4) des Rückhalteteils (2) eine im wesentlichen dreieckige Form aufweist, die in Richtung der Entfernung der Achse (1b) von der Stange (1) zugespitzt ist.

3. Vorrichtung nach einem der Ansprüche 1 oder 2, dadurch gekennzeichnet, daß die Stange (1) und der Rückhalteteil (2) in einem Stück aus einem flexiblen und elastischen, beispielsweise zur Familie der Polyäthylene gehörenden, Material gegossen sind.

4. Vorrichtung nach einem der Ansprüche 1 bis 3, dadurch gekennzeichnet, daß die Stange (1) eine derartige Länge aufweist, daß sich diese Stange (1), wenn die Vorrichtung in der Gebärmutterhöhle (7) angeordnet ist, zur Gänze in dieser Höhle befindet.

5. Vorrichtung nach Anspruch 1, dadurch gekennzeichnet, daß ein Wirkstoff in der Stange (1) inkorporiert ist.

6. Vorrichtung nach Anspruch 5, dadurch gekennzeichnet, daß die Stange (1) hohl und geschlossen ist, sie einen Wirkstoff (8) enthält und ihre Wand ausreichend dünn ist, um den Wirkstoff nach außen diffundieren zu lassen.

7. Vorrichtung nach Anspruch 6, dadurch gekennzeichnet, daß die Stange aus einem verzellten Material hergestellt ist.

## Claims

1. Contraceptive device designed to be placed inside the uterine cavity, of the type comprising an axial stem (1) and a tri-dimensional holding part (2) made of a supple and elastic material, said holding part being secured to one end (1a) of the stem (1) and extending transversely to the axis (1b) of the stem (1) so as to be supported on the lateral uterine walls, said holding part (2) comprising two closed lopps (3, 4) each one of which is secured by way of elastic fastening means to the end (1a) of the stem (1) and being axially symmetrical with respect to the axis of the stem, said loops extending the stem (1) in divergent manner, characterized in that the loops have no symmetry with respect to a plane, but on the contrary are offset laterally in opposite directions one with respect to the other and also with respect to the axis of the stem, as a result of which the said holding part is adaptable in its two transverse dimnensions (a and b) to the dimensions of the uterine cavity, the adjustment of the transverse dimensions (a) of the device to fit the uterine dimensions being made by a partial oberlapping of the loops in the manner of scissors blades.

2. Device according to claim 1, characterized in that each loop (3 and 4) of the holding part (2) has a substabtially triangular shape narrowing down in the direction departing from the axis (1b) and from the stem (1).

3. Device according to any one of claims 1 or 2, characterized in that the stem (1) and the holding part (2) are molded in one piece in a supple and elastic plastic material such as for exemple a material from the polyethylene family.

4. Device according to any one of claims 1 to 3, characterized in that the length of the stem (1) is such that when the devices has been placed inside the uterine cavity (7), said stem (1) is entirely contained inside said cavity.

5. Device according to claim 1, characterized in that an active product is incorporated in the stem (1).

6. Device according to claim 5, characterized in that the stem (1) is hollow and closed, it contains an active product (8) and its wall is thin enough to allow the active product to diffuse out.

7. Device according to claim 6, characterised in that the stem is made of a cellular material.

0 036 805

Fig-1

Fig-2

Fig-4

Fig-3